(19) Europäisches Patentamt / European Patent Office / Office européen des brevets

(11) **EP 2 710 131 B1**

(12) **FASCICULE DE BREVET EUROPEEN**

(45) Date de publication et mention de la délivrance du brevet:
**27.02.2019 Bulletin 2019/09**

(51) Int Cl.:
*C12P 5/00* [(2006.01)]   *C12P 5/02* [(2006.01)]
*C12N 1/12* [(2006.01)]

(21) Numéro de dépôt: **12722715.5**

(22) Date de dépôt: **18.05.2012**

(86) Numéro de dépôt international:
**PCT/EP2012/059230**

(87) Numéro de publication internationale:
**WO 2012/159979 (29.11.2012 Gazette 2012/48)**

(54) **PROCEDE DE PREPARATION ET D'EXTRACTION DU SQUALENE A PARTIR DE MICROALGUES**

VERFAHREN ZUR HERSTELLUNG UND EXTRAKTION VON SQUALEN AUS MIKROALGEN

METHOD OF PREPARATION AND EXTRACTION OF SQUALENE FROM MICROALGAE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.05.2011 CN 201110147052**

(43) Date de publication de la demande:
**26.03.2014 Bulletin 2014/13**

(73) Titulaire: **Roquette Frères
62136 Lestrem (FR)**

(72) Inventeurs:
• **PORA, Bernard
Dongfeng Sunshine City
Wuhan (Economic Develpt Area) 430056 (CN)**
• **QIAN, Yun
Wuhan 430056 (CN)**
• **CAULIER, Bernard
F-59273 Fretin (FR)**
• **COMINI, Serge
F-59253 La Gorgue (FR)**
• **LOOTEN, Philippe
F-59160 Lomme (FR)**
• **SEGUEILHA, Laurent
F-59350 St Andre Lez Lille (FR)**

(74) Mandataire: **Cabinet Plasseraud
66, rue de la Chaussée d'Antin
75440 Paris Cedex 09 (FR)**

• **CHEN G ET AL: "Optimization of nitrogen source for enhanced production of squalene from thraustochytrid Aurantiochytrium sp", NEW BIOTECHNOLOGY, ELSEVIER BV, NL, vol. 27, no. 4, 30 septembre 2010 (2010-09-30), pages 382-389, XP027171921, ISSN: 1871-6784 [extrait le 2010-04-20]**
• **KING WAI FAN ET AL: "Enhanced production of squalene in the thraustochytrid Aurantiochytrium mangrovei by medium optimization and treatment with terbinafine", WORLD JOURNAL OF MICROBIOLOGY AND BIOTECHNOLOGY, KLUWER ACADEMIC PUBLISHERS, DO, vol. 26, no. 7, 8 janvier 2010 (2010-01-08), pages 1303-1309, XP019832729, ISSN: 1573-0972**
• **YUE C J ET AL: "Impact of methyl jasmonate on squalene biosynthesis in microalga Schizochytrium mangrovei", PROCESS BIOCHEMISTRY, ELSEVIER, NL, vol. 44, no. 8, 1 août 2009 (2009-08-01), pages 923-927, XP026160671, ISSN: 1359-5113, DOI: 10.1016/J.PROCBIO.2009.03.016 [extrait le 2009-04-05]**
• **KAYA KUNIMITSU ET AL: "Thraustochytrid Aurantiochytrium sp 18W-13a Accummulates High Amounts of Squalene", BIOSCIENCE BIOTECHNOLOGY AND BIOCHEMISTRY, vol. 75, no. 11, novembre 2011 (2011-11), pages 2246-2248, XP002678626,**

(56) Documents cités:
**WO-A1-2007/039149   WO-A2-2010/023551
US-A- 5 130 242   US-B1- 6 248 779**

- NAKAZAWA ATSUSHI ET AL: "Optimization of culture conditions of the thraustochytrid Aurantiochytrium sp. strain 18W-13a for squalene production", BIORESOURCE TECHNOLOGY, vol. 109, avril 2012 (2012-04), pages 287-291, XP002678627,
- LU-JING REN ET AL: "Development of a stepwise aeration control strategy for efficient docosahexaenoic acid production by Schizochytrium sp", APPLIED MICROBIOLOGY AND BIOTECHNOLOGY, SPRINGER, BERLIN, DE, vol. 87, no. 5, 6 May 2010 (2010-05-06), pages 1649-1656, XP019841637, ISSN: 1432-0614
- LEWIS ET AL.: MAR. BIOTECHNOL., 2001, pages 439-447,
- JIANG ET AL.: J. AGRIC. FOOD CHEM., vol. 52, 2004, pages 1196-1200,

**Description**

La présente invention se rapporte à un procédé de production optimisée de squalène par voie fermentaire à partir de microalgues de la famille des Thraustochytriales sp.

On entend au sens de l'invention par « microalgues de la famille des Thraustochytriales sp. » des microalgues appartenant aux espèces *Schizochytrium sp., Aurantiochytrium sp.* et *Thraustochytrium sp.*

Le squalène est un triterpène, isoprénoide à trente atomes de carbone et cinquante atomes d'hydrogène, de formule : 2,6,10,15,19,23-Hexaméthyl-2,6,10,14,18,22-tétracosahexène.

C'est un lipide naturellement produit par tous les organismes supérieurs y compris chez l'Homme (retrouvé dans le sébum). Le squalène est en effet un intermédiaire essentiel dans la biosynthèse du cholestérol, des hormones stéroïdes et de la vitamine D (une enzyme des voies métaboliques du cholestérol, la squalène monooxygenase, en oxydant l'une des extrémités de la molécule de squalène, va induire sa cyclisation et conduire au lanostérol, lequel sera transformé en cholestérol et en autres stéroïdes).

En industrie, le squalène est surtout utilisé dans les domaines alimentaire, cosmétique et pharmaceutique.

Comme complément alimentaire, le squalène est habituellement formulé en capsules ou en huiles.

Dans le domaine cosmétique, cette molécule peut être utilisée comme antioxydant, antistatique et émollient dans les crèmes hydratantes, pénétrant rapidement la peau sans laisser de traces ou de sensations grasses, et se mélangeant bien avec d'autres huiles et vitamines.

Dans ce domaine, notons qu'étant donné la très grande instabilité du squalène (6 insaturations), c'est la forme saturée squalane (obtenue par hydrogénation), meilleur anti-oxydant que le squalène, que l'on trouve sur le marché, et à niveau de pureté en général très élevé (99%).

Les études toxicologiques ont montré qu'aux concentrations utilisées dans les cosmétiques, le squalène et le squalane ne présentent pas de toxicité, et ne sont pas irritants ou sensibilisants pour la peau humaine.

Dans le domaine pharmaceutique, le squalène est utilisé comme adjuvants aux vaccins.

Ces adjuvants sont des substances qui stimulent le système immunitaire et augmentent la réponse au vaccin.

Sous la forme d'une émulsion ajoutée à la substance vaccinante pour rendre le vaccin plus immunogène, du squalène est utilisé depuis 1997 dans un vaccin antigrippal (*FLUAD*, de la société CHIRON, contre la grippe saisonnière) à raison d'environ 10 mg de squalène par dose.

Comme tous les vaccins contenant du squalène, ces émulsions ont un aspect blanc laiteux.

Le squalène est également utilisé comme adjuvant vaccinal, notamment des vaccins expérimentaux, substances antipaludéennes ou vaccin antigrippe ciblant les virus émergents H5N1 puis en 2009 H1N1, en tant que :

- constituant breveté du système adjuvant AS03 utilisé par la société GLAXOSMITHKLINE dans le vaccin contre la pandémie grippale 2009 Pandemrix et Arepanrix,
- constituant breveté du système adjuvant MF59 utilisé par la société NOVARTIS.

Le squalène a été également ajouté aux vaccins antigrippaux pour stimuler la réponse immunitaire du corps humain à travers la production de cellules CD4 à mémoire.

C'est le premier adjuvant huile-dans-l'eau pour vaccin contre la grippe à avoir été commercialisé en association avec les antigènes du virus de la grippe saisonnière.

Le niveau de pureté du squalène est essentiel dans ce domaine d'applications.

En effet, s'il est pris par voie orale, le squalène est considéré comme totalement sûr ; mais c'est la voie injectable qui fait l'objet de controverses.

En effet, dans le domaine médical, le risque de préjudice pour un receveur humain peut être accru dans les situations où le squalène est contaminé par des impuretés, car par définition, cet adjuvant peut induire une forte réponse immunitaire également contre ses propres impuretés.

Il est donc indispensable de disposer de squalène de haute qualité, exempt d'impuretés (traces de métaux, notamment de mercure, et d'autres toxines).

Un certain nombre de voies de production du squalène sont proposées dans la littérature.

C'est un composé que l'on trouve souvent stocké dans le foie des poissons cartilagineux tels que les requins des profondeurs (d'où son nom).

Il est donc une des causes de leur surpêche, le requin étant déjà chassé pour ses ailerons. Les foies de requin sont ainsi désormais vendus pour produire des gélules qualifiées de "bonnes pour la santé".

Cependant, si le squalène commercialisé est ainsi principalement extrait de foies de requins, il n'est pas exempt de problème sanitaire.

En effet, les requins peuvent être infectés par des pathogènes pouvant produire des substances nuisibles pour l'homme. En outre, le foie des requins, organe d'élimination et de purification de l'organisme, peut contenir des toxines telles que la carchatoxine qui est néfaste pour l'homme.

Ces préoccupations environnementales (forte régression des requins) et sanitaires (le foie des poissons stocke aussi des toxines préoccupantes pour la santé) ont motivé son extraction à partir de végétaux.

Il est ainsi possible de l'isoler de l'huile d'olive, l'huile de palme, et dans d'autres huiles céréalières ou provenant de l'amarante, des semences, du son de riz, de germes de blé.

Cependant, l'inconvénient majeur est ici que le squalène est extrait en très faibles quantités, de l'ordre de 0,1 à 0,7 % en poids.

En première alternative à ces procédés d'extraction à partir de foies de requins ou de végétaux, souvent rendus coûteux par la mise en oeuvre de procédés d'enrichissement et de purification importants, ont été proposés de premiers procédés de production de squalène à partir de microorganismes : levures naturelles ou levures recombinantes, notamment de type *Saccharomyces.*

C'est ainsi que Saccharomyces cerevisiae est connue pour sa capacité à produire du squalène, cependant en très faible quantité : de l'ordre de 0,041 mg/g de biomasse (BHATTACHARJEE, P. et al, 2001, dans World J. Microb. Biotechnol., 17, pp 811-816).

L'optimisation de ces capacités de production a donc été travaillée, par le biais de la recombinaison génétique.

Les levures recombinantes qui produisent du squalène ont ainsi l'avantage :

- de bénéficier du même statut GRAS (*Generally Regarded As Safe*) que la cellule hôte,
- d'être exemptes de pathogènes, de prions ou de toxines, tout comme la cellule hôte, et
- d'avoir déjà été utilisées dans le domaine des vaccins (telles que ces levures qui expriment des vecteurs contenant des antigènes de l'hépatite B).

Cependant, comme le présente la demande de brevet WO 2010/023551 pour le domaine médical (production de squalène d'une pureté supérieure à 97 % comme adjuvant de vaccins), cette première alternative n'est industrialisable que si l'on peut disposer de levures recombinantes hyperproductrices de squalène (à plus de 15 % en poids de cellules sèches).

Or, l'obtention de ces cellules recombinantes nécessite la mise en oeuvre de nombreuses étapes lourdes, longues et complexes d'ingénierie métabolique, par la mise en oeuvre d'outils de biologie moléculaire, conduisant à la stimulation des voies de biosynthèse du squalène et à l'inhibition des voies du catabolisme du squalène.

En effet, comme le rappelle d'ailleurs ladite demande de brevet WO 2010/023551, les gènes impliqués dans la biosynthèse du squalène sont multiples : incluant la mévalonate kinase, la phosphomevalonate kinase, la pyrophosphomevalonate décarboxylase, l'isopentenyl pyrophosphate isomérase, la HMGR (3-hydroxy-3-methylglutaryl-CoA réductase), et la squalène synthétase.

Pour les voies du catabolisme, les gènes codant pour de nombreuses enzymes impliquées dans la conversion du squalène en ergostérol incluant la squalène époxidase (ERGI), la lanostérol synthétase, la C14-diméthylase, la dl4-réductase, la C4-méthyloxidase, la C4-décarboxylase (ERG26), la 3-cétoréductase, la C24-méthyltransférase, la C8-isomérase, la C5-désaturase, la d22-désaturase et la d24-réductase.

Par ailleurs, d'autres enzymes du catabolisme doivent être également considérées : la LEU2 ([beta]-isopropylmalate déshydrogénase), l'oxydosqualène cyclase, la zymostérol-24-méthyltransférase et l'ergosta-5,7,24(28)-trienol-22-déshydrogénase.

En deuxième alternative aux procédés d'extraction à partir de foies de requins ou de végétaux, ont été proposés des procédés prometteurs de production de squalène à partir de microalgues de la famille des Thraustochytriales (comprenant les genres *Thraustochytrium, Aurantiochytrium* et *Schizochytrium*), plus particulièrement *Schizochytrium mangrovei* ou *Schizochytrium limacinum.*

Ces microalgues produisent du squalène en conditions hétérotrophiques (absence de lumière ; apport de glucose comme source carbonée), et peuvent donc être manipulées aisément par l'homme du métier du domaine de la fermentation de microorganismes.

Ces procédés offrent donc, par le biais de conditions de fermentation contrôlées, des qualités de squalène dont la purification est aisément réalisable pour répondre aux besoins alimentaires, cosmétiques et médicales.

Chez ces microalgues de la famille des Thraustochytriales, le squalène est cependant le coproduit d'autres composés lipidiques d'intérêt, tel l'acide docosahexaénoïque (ou DHA), acide gras polyinsaturé de la famille des ω3.

Il apparaît ainsi que le squalène est surtout décrit comme l'un des composants de la fraction insaponifiable des huiles commerciales de DHA (à côté des caroténoïdes et des stérols).

A titre de comparaison, la souche de *Schizochytrium mangrovei* FB1 produit du DHA à raison de 6,2 % en poids sec de cellules, pour 0,017 % de squalène.

De ce fait, ces microorganismes qui produisent naturellement du squalène le font en faibles quantités :

- de l'ordre de 0,1 mg/g de biomasse, pour Thraustochytrid ACEM 6063 (cf. LEWIS et al dans Mar. Biotechnol., 2001, 439-447),

- de l'ordre de 0,162 mg/g de biomasse, pour *Schizochytrium mangrovei* FB1 (cf. JIANG et al, dans J. Agric. Food Chem., 2004, 52, pp 1196-1200)

Pour augmenter ces productions, il est alors apparu indispensable d'optimiser les conditions de fermentation.

Dans l'article de QIAN Li et al, dans J. Agric. Food Chem., 2009, 57, 4267-4272, il est précisé que le squalène est un intermédiaire clef de la biosynthèse des stérols, et que la première étape de la conversion du squalène en stérols est catalysée par une squalène époxidase, dépendante de l'oxygène.

Des conditions riches en oxygène dissous sont donc à proscrire si l'on souhaite au contraire accumuler le squalène intracellulaire.

C'est ainsi que le fait de cultiver Thraustochytride ACEM 6063 à niveau faible en oxygène dissous (0 à 5% de saturation) permet d'accumuler plus de 1 mg/g de squalène, alors que la croissance à plus haut niveau d'oxygène dissous (40 à 60 %) ne permet d'atteindre que 0,01 mg/g de squalène.

De la même manière, une culture à température de 15°C place la production de squalène par Thraustochytride ACEM 6063 à 1,2 mg/g, alors qu'elle n'est que de 0,7 mg/g à 20°C (cf. LEWIS et al, dans Mar. Biotechnol., 2001, 3, 439-447).

Dans l'article de G. CHEN et al., dans New Biotechnology, 2010, 27-4, pp 382-389, il est rappelé que *Schizochytrium* produit principalement du DHA, au travers de la voie des polykétides synthétases (acronyme : PKS), tandis que le squalène est plutôt synthétisé au travers de la voie de biosynthèse du cholestérol, ce qui signifie que les besoins nutritionnels des thrautochytrides pour ces deux composés sont distincts.

L'objet de leur travail a donc été de rechercher de manière systématique l'effet de différentes sources d'azote dans la production de squalène.

G. CHEN et al ont ainsi trouvé que *Schizochytrium* pouvait croître rapidement et accumuler des quantité "élevées" en squalène dans un milieu de culture contenant un mélange de sources azotées constitué de glutamate monosodique, d'extrait de levures et de tryptone.

Malgré cela, cette production "élevée" en squalène est toute relative.

En effet, si les auteurs réussissent à augmenter significativement le contenu en squalène et le rendement de 26,3 % et 10,1 %, respectivement, par rapport aux valeurs du milieu de base, ces conditions optimisées conduisent en fait à un contenu en squalène de 0,72 mg/g et un titre de 5,90 mg/l.

Avec ce même objectif d'optimiser la production de squalène, K. W. FAN et al, dans World J. Microbiol. Biotechnol., 2010, 26-3, pp 1303-1309, ont utilisé un inhibiteur de la squalène monooxygénase (enzyme clef de la biosynthèse des stérols) : la terbinafine hydroxychloride.

Il est connu que le contenu et le rendement en squalène sont liés à l'âge de la culture des microorganismes.

Plus la culture cellulaire vieillit, moins elle accumule du squalène ; en fait plus elle en consomme dans la voie de biosynthèse des stérols.

La terbinafine agit donc en empêchant cette consommation du squalène vers la voie des stérols et permet alors de stimuler l'accumulation intracellulaire de squalène jusqu'à 36 à 40 % par rapport au contrôle.

Mais la production la plus élevée obtenue en squalène avec la souche d'*Aurantiochytrium mangrovei* FB3 utilisée dans cette étude, même si bien supérieure à celle décrite pour *S. cerevisiae* (0,041 mg/g de biomasse), voire même celle décrite pour *Torulaspora debrueckii* (0,24 mg/g de biomasse), n'est que de 0,53 mg/g de biomasse.

Par ailleurs, si cette déviation des voies métaboliques permet la production de squalène en relative plus grande quantité, elle risque de fragiliser les cellules en limitant d'autant la production des stérols indispensables à la fabrication des membranes lipidiques de ces mêmes cellules.

Parmi les résultats les plus élevés de production de squalène rapportés dans la littérature par des microalgues, on trouve dans l'article de C-J YUE et Y. JIANG, Process Biochemisty, 2009, 44, 923-927, un contenu maximal en squalène de $1,17 \pm 0,6$ mg/g de biomasse de *Schizochytrium mangrovei,* qui utilise le méthyle jasmonate pour moduler les voies métaboliques de synthèse du squalène, en agissant directement sur la squalène synthétase, enzyme clef desdites voies métaboliques.

Ainsi, malgré tous les efforts déployés, ces valeurs sont bien inférieures à celles de référence pour l'huile d'olive (de l'ordre de 4,24 mg/g) et sont éloignées des valeurs requises à l'échelle industrielle.

**Soucieuse de mettre au point un procédé de production bien plus efficace et bien moins couteux que ceux décrits dans l'état de la technique,** la société Demanderesse a développé ses propres recherches sur l'optimisation des conditions de fermentation des microalgues de la famille des Thraustochytriales sp.

L'invention concerne donc un procédé permettant l'obtention de squalène à l'échelle du gramme pour 100 grammes de biomasse sèche, soit jusqu'à 1000 fois plus que ce qui décrit habituellement dans la littérature dans ce domaine.

L'invention est également relative à un procédé d'extraction et de purification de squalène à partir du milieu de fermentation ainsi obtenu.

**Production de squalène par fermentation de Schizochytrium**

Il est tout d'abord du mérite de la Société Demanderesse d'avoir trouvé que parmi tous les paramètres de contrôle de la fermentation, deux d'entre eux permettent à eux seuls d'augmenter considérablement le taux de production de squalène chez ces microalgues.

Ces deux paramètres clefs sont la température du milieu de culture, et l'ajout de vitamines, plus précisément des vitamines B1, B6 et surtout de la vitamine B12.

La présente invention se rapporte donc à un procédé de production de squalène par des microalgues appartenant à la famille des Thraustochytriales sp., caractérisé en ce qu'il comprend les étapes suivantes :

- cultiver des microalgues appartenant à la famille des Thraustochytriales sp. à une température comprise entre 25 et 35°C de préférence comprise entre 28 et 32°C, plus préférentiellement de l'ordre de 30°C et
- ajouter dans le milieu de culture 1 à 1000 $\mu$g de vitamine B12 par litre de milieu de culture.

De manière préférentielle, comme il sera indiqué ci-après, la société Demanderesse recommande d'ajouter :

○ 0,1 mg à 200 mg de vitamine B1 par litre de milieu de culture, et/ou
○ 0,1 mg à 200 mg de vitamine B6 par litre de milieu de culture.

Bien entendu, le procédé peut comprendre une étape de récupération de la biomasse riche en squalène et/ou une étape de récupération ou d'extraction du squalène.

Plus particulièrement les souches commercialisées suivantes ont été testées :

- *Schizochytrium sp.* référencée ATCC 20888,
- *Aurantiochytrium sp.* référencée ATCC PRA 276,

Par ailleurs, la société Demanderesse dispose également de sa propre souche de production, une *Schizochytrium sp.* déposée le 14 avril 2011 en France auprès de la Collection Nationale de Cultures de Microorganismes de l'Institut Pasteur sous le n°CNCM I-4469 et également déposée en Chine auprès du CHINA CENTER FOR TYPE CULTURE COLLECTION de l'université de Wuhan, Wuhan 430072, P.R. China sous le n° M 209118.

Une souche de *Schizochytrium mangrovei* a également été testée en témoin, comme il sera exemplifié ci-après.

En particulier, le procédé selon la présente invention permet l'obtention de teneurs en squalène supérieures ou égales à 2 g pour 100 g de biomasse sèche, de préférence comprises entre 2 et 12 g pour 100 g de biomasse sèche. En particulier, la quantification de squalène produit peut être réalisée selon la méthode détaillée dans la partie expérimentale.

Dans le procédé conforme à l'invention, la première caractéristique essentielle est donc le choix de la température à laquelle sera à la fois conduite la culture de la microalgue et sa production en squalène.

La température est ainsi choisie comprise entre 25 et 35°C, de préférence comprise entre 28 et 32°C, plus préférentiellement de l'ordre de 30°C.

La société Demanderesse a donc vaincu un premier préjugé technique qui veut que la culture de ces microalgues ne doit pas dépasser les 25°C.

En effet, dans la plupart des articles cités ci-dessus pour la production de squalène chez les Thraustochytriales, les températures de production étaient réglées sur les températures de croissances des microalgues étudiées, i.e. 15°C, 22°C ou 25°C.

Suivant le procédé de l'invention, la société Demanderesse recommande au contraire de cultiver ces microalgues à 28 et 32°C, car elle a pu noter que :

- si la concentration en squalène produite par la biomasse cellulaire augmente avec la température jusque 33°C, audelà de 30°C la quantité de biomasse se réduit significativement, limitant ainsi le titre en squalène,
- à une température de 25°C, les concentrations en squalène sont indétectables ou très faibles.

Cet intervalle de température est donc un compromis entre la température de l'optimum de culture de la microalgue, et celle de production efficace en squalène.

La température d'au plus 25°C n'est donc pas, contrairement à ce qui est couramment mis en oeuvre dans l'état de la technique, la température favorisant au mieux la production de squalène.

Dans le procédé conforme à l'invention la seconde caractéristique essentielle est la quantité de vitamine B12 apportée au milieu de culture de *Schizochytrium* pour la production de squalène, i.e. à raison de 1 à 1000 $\mu$g de vitamine B12 par litre de milieu de culture.

De manière préférentielle, cet ajout en vitamine B12 peut être complété par :

  ∘ 0,1 mg à 200 mg de vitamine B1 par litre de milieu de culture, et/ou
  ∘ 0,1 mg à 200 mg de vitamine B6 par litre de milieu de culture.

Dans la littérature relative à la production de squalène par les microalgues citée *supra*, le rôle des vitamines n'est pas considéré. L'apport en vitamines se fait classiquement par l'intermédiaire de l'ajout d'extraits de levures.
Il est en effet bien connu de l'homme du métier que ces vitamines sont naturellement présentes dans les extraits de levures à raison de :

- 50 à 120 mg/kg de vitamine B1 (thiamine),
- 40 à 80 mg/kg de vitamine B6 (pyridoxine),
- 1 à 5,5 $\mu$g/kg de vitamine B12 (cyanocobalamine),

pour ne citer que ces trois vitamines B.
Cependant, l'apport en extraits de levures introduits dans les milieux de culture n'est que de 1 à 2 g pour 100 ml de milieu de culture (cf. les articles scientifiques énumérés ci-avant), ce qui correspond à des doses de vitamines extrêmement faibles (par exemple, pour la vitamine B12, cet apport de vitamine B12 par les extraits de levure correspond à 0,07 $\mu$g/l).
Quoi qu'il en soit, l'ensemble de ces documents ne décrit ni ne suggère le contrôle de la teneur en vitamines B1, B6 ou B12 pour la production de squalène chez les microalgues.
Sans être liée par une quelconque théorie, la société Demanderesse a trouvé que le rôle prépondérant de la vitamine B12 dans la production de squalène (comme il sera exemplifié ci-après) suggèrerait son implication comme cofacteur de certaines des enzymes clefs impliquées dans la biosynthèse du squalène.
Quant à la vitamine B1, elle stimulerait la voie de dégradation de la leucine, ce qui augmenterait la quantité intracellulaire des précurseurs du squalène, et la vitamine B6, en modifiant l'action des cytochromes, éviterait la dégradation du squalène.
La société Demanderesse a donc trouvé que l'apport de vitamine B12 est, avec la température amenée à 28 et 30°C, la clef pour augmenter considérablement la production de squalène (à l'échelle du g/100 g de biomasse sèche), et celui des vitamines B1 et B6 permet en fait surtout d'augmenter la productivité en squalène, comme il sera démontré ci-après.
Une autre caractéristique du procédé conforme à l'invention est que l'ajout des vitamines peut être réalisé tout le long du procédé de fermentation, ou au niveau de certaines de ses étapes, et pas seulement en phase de production.
Il faut cependant respecter l'apport compris entre 1 et 1000 $\mu$g/l de vitamines B12 sur l'ensemble du procédé de fermentation.
Classiquement, dans la littérature relative aux travaux d'optimisation des conditions de production de squalène par microalgue réalisés au laboratoire, la fermentation est menée sur la base d'une chaîne d'ensemencement et de production microbienne classique, et ce sont les conditions de production qui sont généralement les plus travaillées.
La production de squalène à partir de *Thraustochytrium* demande en effet trois étapes successives : partir d'une colonie isolée sur boite gélosée, préculture pour revivifier la souche et enfin culture (= production) proprement dite.
Par exemple dans l'article de G. CHEN cité *supra*, on trouve le procédé qui comprend les étapes successives suivantes :

- partir d'une souche maintenue sur milieu nutritif gélosée comprenant du glucose, du glutamate monosodique, de l'extrait de levures et des oligoéléments divers,
- réaliser une préculture en Erlenmeyers sur agitateur orbital, à un pH de 6, à une température de 25°C afin d'obtenir une biomasse revivifiée,
- ensemencer une autre série d'Erlenmeyers de production, avec le même milieu de culture que celui utilisé en préculture, avec de l'ordre de 0,5 % (v/v) de la biomasse obtenue à l'étape précédente, et maintenir la température à 25°C.

Or, comme on peut le lire dans cet article (mais cela se vérifie pour les autres articles du domaine), c'est au niveau de cette dernière étape de culture que les spécialistes agissent pour optimiser les conditions de fermentation.
En d'autres termes, les études d'optimisation de l'état de la technique consistent à faire varier un ou plusieurs composants du milieu de production afin d'en étudier l'influence sur la production de squalène.
Or, comme il sera développé ci-après, la société Demanderesse recommande au contraire de contrôler les conditions de fermentation dès les premières étapes, même si celles-ci se révèlent plus complexes à mettre en oeuvre à l'échelle industrielle.

A cette échelle en effet, la chaîne d'ensemencement peut être composée de plusieurs étapes en série de préculture avant l'étape de production proprement dite.

Un mode de réalisation préféré du procédé conforme à l'invention peut alors consister en la succession des étapes suivantes :

- première préculture de la microalgue de la famille des Thraustochytriales pendant 24 à 36 heures en Erlenmeyers, à une température de 28°C à partir d'une colonie isolée sur boite gélosée,
- seconde préculture pendant 24 à 36 heures en Erlenmeyers, à une température de 28°C avec un inoculum de 1 % (v/v) issu de la première préculture,
- culture pendant 60 à 150 heures à 30°C, en fermenteur conditionné de manière à respecter un transfert d'oxygène d'au plus 45 mmoles/l/heure, avec un inoculum de 0,5 à 2 % (v/v) issu de la seconde préculture.

Dans ce chaînage, en fonctions des conditions opératoires, comme il sera exemplifié ci-après, l'ajout des vitamines peut se faire dans une seule étape de préculture, dans deux étapes de préculture ou tout le long de la chaîne de culture.

Comme il sera démontré ci-après ; l'ajout de vitamines dans une seule étape de préculture permet déjà d'obtenir de remarquables résultats, le mode optimisé demandant un ajout à toutes les étapes mises en oeuvre.

La source carbonée nécessaire à la croissance de la microalgue est préférentiellement du glucose.

La société Demanderesse recommande alors de contrôler l'ajout de glucose de manière à apporter une quantité totale de glucose de 15 à 22,5 % en poids.

Quoi qu'il en soit, comme il sera exemplifié ci-après avec les souches de *Schizochytrium* choisies, il est préféré de travailler à une teneur en glucose résiduel non nulle, au plus égale à 8 % en poids.

Quant à la nature de la source d'azote, la société Demanderesse a trouvé qu'il est possible de la choisir dans le groupe constitué des extraits de levure, de l'urée, du glutamate de sodium, du sulfate d'ammonium pris seuls ou en combinaison.

Il est possible de préférer aux extraits de levure, classiquement mis en oeuvre dans les procédés de l'état de la technique, de l'urée complétée par un cocktail de vitamines, telle le cocktail BME commercialisé par la société SIGMA, utilisé à raison de 5 ml/l.

De la même façon, il est possible de remplacer l'urée par du glutamate de sodium en tout ou partie, ou utiliser un mélange de glutamate de sodium et de sulfate d'ammonium.

La société Demanderesse recommande surtout de ne pas utiliser de source d'azote sous formes nitriques.

Quant au pH du milieu de culture, comme il sera exemplifié ci-après, il sera maintenu entre 5,5 et 6,5, préférentiellement fixé à une valeur de 6.

La régulation du pH peut se faire par tout moyen connu de l'homme du métier, par exemple par ajout d'acide sulfurique 2 N, puis avec de la soude 8 N.

Enfin, le taux d'oxygène dissous peut être régulé à une valeur comprise entre 20 et 0 %, de préférence maintenu à 5 % pendant une durée initiale de 24 et 48 heures, de préférence 36 heures, avant d'être laissée à 0 %.

Quant au transfert d'oxygène, il sera régulé par tout moyen connu par ailleurs de l'homme du métier, de manière à ne pas dépasser 45 mmoles/l/heure.

Comme il sera exemplifié ci-après, la biomasse obtenue dans ces conditions opératoires, en fin de fermentation, est de plus de 50 g/l, de préférence comprise entre 50 et 100 g/l, de l'ordre de 80 g/l.

La teneur en squalène est quant à elle de plus de 1 g pour 100 g de biomasse sèche, de préférence comprise entre 2 et 15 g pour 100 g de biomasse sèche, plus préférentiellement encore comprise entre 5 et 10 g pour 100 g de biomasse sèche.

**Extraction et purification du squalène du milieu de fermentation**

La biomasse est récupérée du milieu de fermentation par toute méthode connue en soi de l'homme du métier, par exemple la biomasse peut être extraite du fermenteur et simplement concentrée par microfiltration ou centrifugation, ou lavée par succession de concentrations-dilutions avec une solution aqueuse.

La rupture des cellules pour l'extraction du contenu lipidique peut être effectuée par différentes voies parmi lesquelles les voies mécanique, chimique, enzymatique.

L'huile est extraite du lysat cellulaire à l'hexane/éthanol en plusieurs extractions successives.

La fraction hexanique est séparée puis l'hexane est évaporé pour isoler l'huile brute.

La présente invention concerne finalement l'utilisation du squalène produit par l'un quelconque des procédés de la présente invention dans la préparation de compositions destinées aux domaines médicaux, cosmétiques et alimentaires. Ainsi, elle concerne une méthode de préparation de compositions destinées aux domaines médicaux, cosmétiques et alimentaires comprenant la production de squalène par l'un quelconque des procédés de la présente invention, puis la préparation de compositions destinées aux domaines médicaux, cosmétiques et alimentaires.

L'invention sera mieux comprise à l'aide des exemples qui suivent, lesquels se veulent illustratifs et non limitatifs.

**Exemple 1** : **Etude de l'ajout des vitamines B1, B6 et B12 et de l'influence de la température sur la production en squalène**

Milieux de préculture et culture

La fermentation des microalgues a été conduite ici en deux phases de préculture successives préalables avant la phase de culture / production proprement dite.

Pour cette expérimentation, l'ajout des vitamines a été assuré dans le premier milieu de préculture, mais a été optionnel dans le second milieu de préculture et en production.

Les milieux de préculture présentaient alors la composition présentée dans les tableaux I et II suivants :

Tableau I

| Milieu de la première préculture | % |
|---|---|
| Glucose | 3 |
| Extraits de levure | 0,4 |
| Sodium glutamic acid | 6,42 |
| Na Cl | 1,25 |
| MgSO4 | 0,4 |
| KCl | 0,05 |
| CaCl2 | 0,01 |
| NaHCO3 | 0,05 |
| KH2PO4 | 0,4 |
| Mélange vitamines | 0,14 |
| Oligo-éléments | 0,8 |

Tableau II

| Milieu de la seconde préculture | % |
|---|---|
| Glucose | 8,57 |
| Sodium glutamic acid | 6,42 |
| Extraits de levure | 0,64 |
| Na Cl | 2 |
| KH2PO4 | 0,64 |
| MgSO4 | 2,29 |
| CaCl2 | 0,03 |
| NaHCO3 | 0,03 |
| Na2 SO4 | 0,03 |
| ajout du mélange de vitamines | 0,14 |
| Oligo-éléments | 0,2 |

D'une manière générale on a utilisé de l'anti-mousse Clerol « FBA3107 » à 1 ml/l.

Eventuellement on a utilisé 50 mg /L de Pénicilline G "sodium salt" afin d'éviter la croissance de bactéries contaminantes.

Le glucose a été stérilisé avec le $KH_2PO_4$ et séparément du reste du milieu car on a évité ainsi la formation d'un précipité (Ammonium-Phosphate-Magnésium).

Le mélange des vitamines et les oligo-éléments ont été ajoutés après filtration stérilisante.

La composition du milieu de culture / production est donné par le tableau III suivant.

Tableau III

|  | % |
|---|---|
| Glucose Ajout à T0 | 7,5 |
| Urée | 1 |
| Extraits de levure | 1,2 |
| Na Cl | 0,25 |
| KH2PO4 | 0,96 |
| MgSO4 | 1,2 |
| CaCl2 | 0,12 |
| NaHCO3 | 0,12 |
| KCL | 0,08 |
| Si ajout du mélange de vitamines | 0,4 |
| Oligo-éléments | 0,56 |

La composition des mélanges de vitamines et des oligoéléments est donnée dans les tableaux IV et V suivants :

Tableau IV

| Mélange vitamines | g/L |
|---|---|
| B1 | 45 |
| B6 | 45 |
| B12 | 0.25 |

Tableau V

| Oligo-éléments | g/L |
|---|---|
| MnCl2 2h20 | 8.60 |
| CoCl2 6H2O | 0.2 |
| NiSO4 6H2O | 7.50 |
| Na2MoO4 2H2O | 0.15 |
| ZnSO4 7H2O | 5.70 |
| Cu So4 5h2O | 6.50 |
| FeSO4 7 H2O | 32.00 |
| ZnCl2 | 1.50 |

Conduite de la fermentation

La première préculture a été réalisée en Erlenmeyers de 500 ml munis de baffles, dans lesquels on a ajouté une goutte d'antimousse CLEAROL FBA 3107 commercialisé par la société COGNIS GmbH Düsseldorf.

Le milieu de culture a été filtré après dissolution complète de ses constituants, complété éventuellement avec de la pénicilline G "sodium salt" à raison de 0,25 mg/l.

L'inoculation a été réalisée par prélèvement de colonies de microalgues cultivées en boite de Pétri (à raison d'une oese de 10 $\mu$l).

L'incubation a duré 24 à 36 heures, à une température de 28°C, sous agitation à 100 rpm (sur agitateur orbital).

La biomasse décantant (ou adhérant à la paroi), on a pris bien soin de prélever 3 à 5 ml après avoir bien agité l'Erlenmeyer.

Pour la seconde préculture, on a utilisé des Erlenmeyers de 2 l, muni de baffles et de tuyauterie.

On a ajouté une goutte d'antimousse et l'extrait de levures dans 100 ml d'eau.

L'ensemble des constituants du milieu a été filtré après dissolution dans 300 ml d'eau déminéralisée. On pouvait éventuellement ajouter de la pénicilline G "sodium salt" et au préalable dans l'Erlenmeyer une goutte d'antimousse avant sa stérilisation.

L'ensemencement s'est fait ensuite avec 3 à 5 ml de la première préculture.

L'incubation a été réalisée à 28°C pendant encore 24 à 36 heures, sous agitation à 100 rpm.

La culture proprement dite a été réalisée de la manière suivante en réacteur de 20 l.

- stérilisation du milieu pour partie dans le réacteur, et séparément pour l'autre partie de manière à éviter la formation d'un précipité,
- ensemencement réalisé à partir de la biomasse produite en fin de seconde préculture à raison de 0,5 % v/v du milieu de culture,
- culture maintenue à 30°C
- taux de transfert d'oxygène fixé à 35 - 40 mmoles/l/h,
- aération de 0,2 à 0,3 WM,
- pH initial > 5,5.
- alimentation du glucose dès que la concentration est > 20 %, de manière à maintenir une concentration en glucose comprise entre 15 et 70 g/l.

Le tableau suivant présente les résultats obtenus la *Schizochytrium sp.* de la société Demanderesse.

Ce tableau présente :

- l'effet de la température sur la production de squalène (ajout de vitamines uniquement dans les deux précultures : essais « B » et « C »), et
- l'effet de l'ajout des vitamines B1, B6 et B12 dans les étapes de précultures et de production (essais « D » et « E »), par rapport à un standard sans ajout de vitamines (essai « A »).

Un témoin sans ajout complémentaire de vitamines à 25°C a été également réalisé.

Tableau V :

| Essais | A | B | C | D | E |
|---|---|---|---|---|---|
| Ajout des vitamines B1, B6 et B12 en précultures | Non | Oui | | Oui | |
| Ajout de vitamines B1, B6 et B12 en production | Non | Non | | Oui | |
| Température des précultures (°C) | 25 | 28 | 28 | 28 | 28 |
| Température de culture (C) | 25 | 25 | 30 | 25 | 30 |
| Titre en squalène en fin de culture (g/l) | Non Détectable (ND) | 0,7 | 0,5 | 2,3 | 4,4 |
| Biomasse (g/l) | 35 | 64 | 53 | 70 | 54 |
| % de squalène sur biomasse sèche | ND | 1,1 | 0,9 | 3,3 | 8,2 |

Il est à noter que le témoin "A", dans les conditions classiques de la littérature, n'a pas permis la production de quantités détectables de squalène pour la souche de microalgue testée.

Pour les essais « B » et « C » :

Une température de 28°C en préculture, et la présence des vitamines B1, B6 et B12 en précultures a permis déjà d'assurer une production en squalène de l'ordre de 1 g pour 100 g de biomasse (essais "B"), soit environ 1000 fois plus que ce qui est décrit dans la littérature (par exemple pour la production de squalène par Thraustochytrid ACEM 6063 ou *S. mangrovei* FB1).

Pour les essais « D » et « E » :

L'effet de la température couplée à l'ajout de vitamines à toutes les étapes de culture (les 2 précultures et la production), est remarquable. L'essai "D" a permis la production de 3,3 g de squalène pour 100 g de biomasse lorsque la température a été laissée à 25°C, qui a augmenté pour l'essai "E" à 8,2 g/l lorsque la température a été amenée à 30°C.

La conduite des conditions de fermentation à des températures supérieures à 25°C, couplées à l'ajout conséquent de vitamines B1, B6 et B12 permettent donc d'obtenir les rendements et productivité en squalène les plus importantes.

Méthode de quantification du squalène dans la biomasse de *Schizochytrium sp*.

L'analyse a été réalisée par RMN du proton à 25°C après cassage billes de la biomasse et extraction au chloroforme/méthanol à froid. La quantification a été faite au moyen d'un étalon interne tel que décrit ci-dessous.

Les spectres ont été obtenus sur un spectromètre AVANCE III 400 (Bruker Spectrospin), opérant à 400 MHz.

Cassage de la biomasse : Peser précisément environ 200 mg de biomasse fraîche. Ajouter environ 1-1,5 cm de billes verre et 0,1 mL de Méthanol. Fermer hermétiquement le tube et agiter au moyen de l'agitateur vortex pendant au moins 5 min.

Extraction à froid : Ajouter environ 2 mg de triphényl phosphate (TPP), 0,9 mL de Méthanol et 2 mL de chloroforme. Fermer hermétiquement le tube et agiter au moyen de l'agitateur vortex pendant 1 min. Placer au réfrigérateur. Après décantation (au minimum 1 heure), récupérer délicatement la phase supérieure limpide et la transférer dans un godet verre pour évaporation à sec, à température ambiante, sous courant d'azote. Solubiliser l'extrait sec dans 0,5 mL de $CDCl_3$ et 0,1 mL de $CD_3OD$ et transférer dans un tube RMN.

Enregistrement du spectre : Effectuer l'acquisition, sans suppression de solvant, sans rotation, avec un temps de relaxation d'au moins 15 s, après les réglages appropriés de l'instrument. La fenêtre spectrale doit être au moins comprise entre -1 et 9 ppm en calibrant le spectre sur le pic de chloroforme à 7,25 ppm. Le spectre est exploité après transformation de Fourier, correction de phase et soustraction de la ligne de base en mode manuel (sans multiplication exponentielle, LB=GB=0).

Exploitation du signal : Attribuer la valeur 100 au massif du TPP ne contenant pas le signal du chloroforme entre 7,05 et 7,15 ppm (comptant pour 9 protons TPP). Intégrer la surface du signal Squalène à 1,55 ppm (singulet comptant pour 6 protons). Calcul et expression des résultats : Les résultats ont été exprimés en pourcentage massique brut.

$$\text{Teneur} = \frac{A_s \times P_{TPP}}{6 \times 100} \times \frac{W_{TPP}}{M_{TPP}} \times M_S \times \frac{100}{PE}$$

avec

$A_S$ : surface du signal Squalène à 1,55 ppm.
$P_{TPP}$ : nombre de protons du massif TPP intégré : 9
$W_{TPP}$ : masse, en grammes, de TPP pesé
$M_{TPP}$ : masse molaire, en grammes par mole, du TPP ($M_{TPP}$=326 g/mol)
$M_S$ : masse molaire, en grammes par mole, du Squalène ($M_S$ =410 g/mol)
PE : masse, en grammes, de biomasse fraîche

**Exemple 2 : Etude de l'influence des vitamines B1, B6 et B12**

L'expérimentation menée ici avait pour but de rendre compte de l'importance relative des vitamines dans les rendements et productivité en squalène.

La température des milieux de préculture a été définie, comme dans l'exemple 1, à 28°C, et la température du milieu de culture / production a été maintenue à 30°C.

Deux séries d'expérimentation ont été menées :

- ajout de vitamines B1, B2 et B6 au niveau des différentes étapes de préculture et production,
- rôle de la vitamine B12 seule ou combinée aux vitamines B1 et B6. Les conditions générales de culture étaient celles décrites dans l'exemple 1, avec cependant une modification quant à l'inoculation avec la seconde préculture a été portée à 2 % v/v de la culture.

Le tableau VI suivant rassemble les résultats obtenus avec la souche de *Schizochytrium sp* de la société Demanderesse.

| | F | G | | | H | | | I | | | J | | | K | | |
|---|---|---|---|---|---|---|---|---|---|---|---|---|---|---|---|---|
| Vitamines dans la 1ère préculture (µg/l) | Non | B1 + B6 + B12 | | | B1 + B6 + B12 | | | B1 + B6 + B12 | | | B1 + B6 + B12 | | | B1 + B6 + B12 | | |
| | | 63000 | 63000 | 350 | 63000 | 63000 | 350 | 63000 | 63000 | 350 | 63000 | 63000 | 350 | 63000 | 63000 | 350 |
| Vitamines dans la 2NDE préculture (µg/l) | Non | Non | | | B1 + B6 + B12 | | | B1 + B6 + B12 | | | B12 | | | B1 +B6 + B12 | | |
| | | | | | 63000 | 63000 | 350 | 63000 | 63000 | 350 | | 350 | | 63000 | 63000 | 350 |
| Vitamines en production (µg/l) | Non | Non | | | Non | | | B1 + B6 + B12 | | | B12 | | | B12 | | |
| | | | | | | | | 180000 | 180000 | 1000 | 1000 | | | 1000 | | |
| Titre final (g/l) | ND | 0,2 | | | 3,6 | | | 6,7 | | | 4,9 | | | 7,4 | | |
| Biomasse (g/l) | 35 | 40 | | | 47 | | | 76 | | | 53 | | | 74 | | |
| squalène sur biomasse sèche (% g/g) | ND | 0,4 | | | 7,7 | | | 8,8 | | | 8,9 | | | 10 | | |
| Glucose consommé (g/l) | 135 | 138 | | | 144 | | | 215 | | | 120 | | | 220 | | |

Dans les essais « G », « H » et « I » :

- Pour l'essai « G », il est montré que la production de squalène a atteint déjà 0,4 g pour 100 g de biomasse sèche, i.e. 4 mg / g de biomasse, ce qui est le triple du résultat obtenu dans l'article de C-J YUE et Y. JIANG, Process Biochemisty, 2009, 44, 923-927, i.e. avec un contenu maximal en squalène de 1,17 $\pm$ 0,6 mg/g de biomasse de *Schizochytrium mangrovei* en présence de méthyle jasmonate.
- Ce résultat a atteint des valeurs encore plus remarquable lorsque l'apport en vitamines a été maintenu lors de la seconde préculture (7,7 g pour 100 g de biomasse sèche), pour atteindre une valeur de près de 9 g pour 100 g de biomasse sèche quand cet ajout de vitamines a été reconduit en production.

Quant aux essais « J » et « K », ils démontrent surtout que l'ajout de vitamine B12 est nécessaire et suffisant pour atteindre la valeur des 9 g pour 100 g de biomasse.
Cette valeur a même atteint 10 g pour 100 g de biomasse sèche quand les vitamines B6 et B1 ont été ajoutées dans le deuxième milieu de préculture.
Il est à noter que la différence entre les essais « I » et « J » se joue au niveau du titre en squalène : Pour 9 g de squalène produit pour 100 g de biomasse sèche, le titre a été inférieur dans l'essai "J" par rapport au "I" : les vitamines B1 et B6 ont donc bien permis d'augmenter la productivité du système (par accroissement de la biomasse).

## Exemple 3 : **Essais comparatifs**

Cette expérimentation visait à démontrer que les conditions opératoires testées dans les exemples 1 et 2 peuvent également s'appliquer à d'autres microalgues du type de celle détenue par la société Demanderesse :

- *Schizochytrium sp.* référencée ATCC 20888,
- *Aurantiochytrium sp.* référencée ATCC PRA 276

Les conditions opératoires ont été identiques à l'essai "I" de l'exemple 2.
En témoin : une souche classiquement mise en oeuvre dans la littérature pour sa capacité à produire du squalène : *Schizochytrium mangrovei.*
Le tableau VII suivant présente les résultats obtenus.

Tableau VI

|  | Biomasse (g/l) | % squalène (g/g) | Titre squalène (g/l) |
|---|---|---|---|
| I | 76 | 8,8 | 6,7 |
| *Schizochytrium sp.* ATCC 20888 | 60 | 6,2 | 3,7 |
| *Aurantiochytrium sp.* ATCC PRA 276 | 72 | 2,3 | 1,7 |
| *Schizochytrium mangrovei* | 82 | 0,3 | 0,2 |

La production en squalène a été supérieure à 2 g pour 100 g de biomasse pour toutes les Thraustochytriales de la sous famille *sp* testée. Celle de *S. mangrovei* est conforme à ce qu'il est décrit dans la littérature (pour les meilleurs résultats obtenus) : 2 mg de squalène / g de biomasse sèche.

## Exemple 4 : Obtention de l'huile brute riche en squalène produit par fermentation

La biomasse obtenue au terme de l'exemple 1 (essais « E ») était à une concentration de 54 g/l en fin de fermentation.
Le titre en squalène obtenu en fin de fermentation était de 4,4 g/l.
La biomasse a été extraite du fermenteur puis concentrée par centrifugation à 120 g/l.
La biomasse a été maintenue sous agitation à 150 rpm dans une cuve 50 l, et est chauffée à 60°C.
Le pH a été alors ajusté à 10 à la potasse 45 %.
Ces conditions ont été maintenues pendant 6 h pour parvenir à une lyse alcaline complète.
La qualité de la lyse a été suivie au microscope optique et par centrifugation d'échantillon (2 min, 10000 g).
En fin de lyse, 10 litres d'éthanol (1 volume d'éthanol /volume lysat) ont été ajoutés dans la cuve maintenue à 45°C et agitée pendant 10 min.
10 litres d'hexane ont été ensuite ajoutés dans la cuve maintenue agitée pendant 30 min.

Le mélange a été ensuite centrifugé afin de séparer la fraction légère (hexane + huile) qui a été stockée dans une cuve de 1 m$^3$.

La phase lourde (aqueuse) a été à nouveau mise en présence de 10 litres d'hexane pour procéder à une seconde extraction sur le même schéma que précédemment afin d'augmenter le rendement d'extraction.

Les deux fractions organiques ont été regroupées afin de procéder à l'évaporation de l'hexane en évaporateur rotatif.

Les résidus d'hexane de l'huile extraite ont été éliminés par évaporation à l'évaporateur à film raclé (80°C ; 1mbar).

L'huile brute a été ainsi récupérée avec un rendement de 70%.

## Revendications

1. Procédé de production de squalène par des microalgues appartenant à la famille des Thraustochytriales sp. choisies dans le groupe constitué de *Schizochytrium sp., Aurantiochytrium sp.* et *Thraustochytrium sp*, **caractérisé en ce qu'**il comprend les étapes consistant à :

   - cultiver des microalgues appartenant à la famille des Thraustochytriales sp. choisies dans le groupe constitué de *Schizochytrium sp., Aurantiochytrium sp.* et *Thraustochytrium sp* à une température comprise entre 25 et 35°C, et
   - ajouter dans le milieu de pré-culture ou de culture 1 à 1000 µg de vitamine B12 par litre de milieu de culture.

2. Procédé selon la revendication 1, **caractérisé en ce que** l'on cultive les microalgues à une température comprise entre 28 et 32 °C..

3. Procédé selon la revendication 1 ou 2, **caractérisé en ce que** l'on cultive les microalgues à une température de l'ordre de 30°C.

4. Procédé selon l'une quelconque des revendications 1 à 4, **caractérisé en ce que** l'on ajoute :

   ◦ 0,1 mg à 200 mg de vitamine B1 par litre de milieu de culture, et/ou
   ◦ 0,1 mg à 200 mg de vitamine B6 par litre de milieu de culture.

5. Procédé selon l'une quelconque des revendications 1 à 4, **caractérisé en ce que** les microalgues de la famille de Thraustochytriales sont choisies dans le groupe constitué de *Schizochytrium sp.* ATCC 20888, de *Aurantiochytrium sp.* ATCC PRA 276 et de la souche *Schizochytrium sp.* déposée auprès de la Collection Nationale de Cultures de Microorganismes de l'Institut Pasteur sous le n°CNCM I-4469.

6. Procédé selon l'une quelconque des revendications 1 à 5, **caractérisé en ce que** les teneurs en squalène obtenues sont supérieures ou égales à 2 g pour 100 g de biomasse sèche, de préférence comprise entre 2 g et 12 g pour 100 g de biomasse sèche.

7. Procédé selon l'une quelconque des revendications 1 à 6, **caractérisé en ce que** la culture des microalgues appartenant à la famille des Thraustochytriales sp. est réalisée par la succession des étapes suivantes :

   - première préculture pendant 24 à 36 heures en Erlenmeyers, à une température de 28°C à partir d'une colonie isolée sur boite gélosée,
   - seconde préculture pendant 24 à 36 heures en Erlenmeyers, à une température de 28°C avec un inoculum de 1 % (v/v) issu de la première préculture,
   - culture pendant 60 à 150 heures à 30°C, en fermenteur conditionné de manière à respecter un transfert d'oxygène d'au plus 45 mmoles/l/heure, avec un inoculum de 0,5 à 2 % (v/v) issu de la seconde préculture.

8. Procédé selon la revendication 7, **caractérisée en ce que** l'ajout de vitamines B12 est réalisé au niveau des deux étapes de préculture, de manière à ce que la teneur totale en vitamines soit comprise entre 1 et 10 µg/l de milieu de culture.

9. Procédé selon la revendication 8, **caractérisé en ce que** l'on ajoute également des vitamines B1 et B6 au niveau des deux étapes de préculture, de manière à ce que la teneur totale en vitamines soit comprise entre 100 et 200

$\mu$g/l de milieu de culture.

10. Procédé selon la revendication 7, **caractérisée en ce que** l'ajout de vitamines B12 est réalisé :

   - au niveau des deux étapes de préculture, de manière à ce que la teneur totale en vitamines soit comprise entre 1 et 10 $\mu$g/l de milieu de culture,
   - au niveau de l'étape de production, de manière à ce que la teneur totale en vitamines soit de l'ordre de 1000 $\mu$g/l de milieu de culture.

11. Procédé selon la revendication 10, **caractérisé en ce que** l'on ajoute également des vitamines B1 et B6 :

   - au niveau des deux étapes de préculture, de manière à ce que la teneur totale en vitamines soit comprise entre 100 et 200 $\mu$g/l de milieu de culture,
   - au niveau de l'étape de production, de manière à ce que la teneur totale en vitamines soit comprise entre 150 et 200 mg/l de milieu de culture.

## Patentansprüche

1. Verfahren zur Herstellung von Squalen durch Mikroalgen, die zur Familie der Thraustochytrien sp. gehören, gewählt aus der Gruppe, umfassend *Schizochytrium sp., Aurantiochytrium sp.* und *Thraustochytrium sp.,* **dadurch gekennzeichnet, dass** es die folgenden Schritte umfasst:

   - Kultivieren von Mikroalgen, die zur Familie der Thraustochytrien sp. gehören, gewählt aus der Gruppe, umfassend *Schizochytrium sp., Aurantiochytrium sp.* und *Thraustochytrium sp.,* bei einer Temperatur von 25 bis 35 °C, und
   - Hinzugeben von 1 bis 1000 $\mu$g Vitamin B12 pro Liter Kulturmedium zum Vorkulturmedium oder Kulturmedium.

2. Verfahren nach Anspruch 1, **dadurch gekennzeichnet, dass** die Mikroalgen bei einer Temperatur von 28 bis 32 °C kultiviert werden.

3. Verfahren nach Anspruch 1 oder 2, **dadurch gekennzeichnet, dass** die Mikroalgen bei einer Temperatur im Bereich von 30 °C kultiviert werden.

4. Verfahren nach einem der Ansprüche 1 bis 4, **gekennzeichnet durch** den Zusatz von:

   * 0,1 bis 200 mg Vitamin B1 pro Liter Kulturmedium, und/oder
   * 0,1 bis 200 mg Vitamin B6 pro Liter Kulturmedium.

5. Verfahren nach einem der Ansprüche 1 bis 4, **dadurch gekennzeichnet, dass** die Mikroalgen der Familie der Thraustochytrien gewählt sind aus der Gruppe, umfassend *Schizochytrium sp.* ATCC 20888, *Aurantiochytrium sp.* ATCC PRA 276 und dem Stamm *Schizochytrium sp.,* der unter der Nummer CNCM I-4469 bei der Collection Nationale de Cultures de Microorganismes des Institut Pasteur hinterlegt ist.

6. Verfahren nach einem der Ansprüche 1 bis 5, **dadurch gekennzeichnet, dass** der erhaltene Squalen-Gehalt größer oder gleich 2g pro 100g Biotrockenmasse beträgt, bevorzugt 2 bis 12g pro 100g Biotrockenmasse beträgt.

7. Verfahren nach einem der Ansprüche 1 bis 6, **dadurch gekennzeichnet, dass** die Mikroalgenkultur der Familie der Thraustochytrien sp. durch die Aufeinanderfolge der folgenden Schritte realisiert wird:

   - erste Vorkultur über 24 bis 36 Stunden im Erlenmeyerkolben bei einer Temperatur von 28 °C auf der Basis einer isolierten Kolonie auf einer Gelplatte,
   - zweite Vorkultur über 24 bis 36 Stunden im Erlenmeyerkolben bei einer Temperatur von 28 °C mit einer Impfung von 1 % (v/v) aus der ersten Vorkultur,
   - Kultur über 60 bis 150 Stunden bei 30 °C in einem Fermenter so dass ein Sauerstofftransfer von höchstens 45 mmol/l/Stunde gewährleistet ist, mit einer Impfung von 0,5 bis 2 % (v/v) aus der zweiten Vorkultur.

8. Verfahren nach Anspruch 7, **dadurch gekennzeichnet, dass** der Zusatz von Vitamin B12 in den zwei Vorkulturschritten derart erfolgt, dass der Gesamtvitamingehalt bei 1 bis 10 $\mu$g/l Kulturmedium liegt.

9. Verfahren nach Anspruch 8, **dadurch gekennzeichnet, dass** ferner in den zwei Vorkulturschritten Vitamin B1 und Vitamin B6 zugesetzt werden, derart, dass der Gesamtvitamingehalt bei 100 bis 200 $\mu$g/l Kulturmedium liegt.

10. Verfahren nach Anspruch 7, **dadurch gekennzeichnet, dass** der Zusatz von Vitamin B12 wie folgt realisiert wird:

   - in den zwei Vorkulturschritten derart, dass der Gesamtvitamingehalt bei 1 bis 10 $\mu$g/l Kulturmedium liegt,
   - im Produktionsschritt derart, dass der Gesamtvitamingehalt in der Größenordnung von 1000 $\mu$g/l Kulturmedium liegt.

11. Verfahren nach Anspruch 10, **dadurch gekennzeichnet, dass** ferner Vitamin B1 und Vitamin B6 zugesetzt werden:

   - in den zwei Vorkulturschritten derart, dass der Gesamtvitamingehalt bei 100 bis 200 $\mu$g/l Kulturmedium liegt,
   - im Produktionsschritt derart, dass der Gesamtvitamingehalt bei 150 bis 200 mg/l Kulturmedium liegt.

**Claims**

1. A process for the producing squalene from microalgae belonging to the Thraustochytriales sp. family selected from the group consisting of *Schizochytrium sp., Aurantiochytrium sp.* and *Thraustochytrium sp,* wherein it comprises the steps of :

   - culturing microalgae belonging to the Thraustochytriales sp. family selected from the group consisting of *Schizochytrium sp., Aurantiochytrium sp.* and *Thraustochytrium sp* at a temperature of between 25 and 35°C, and
   - adding to preculture medium or culture medium from 1 to 1000 $\mu$g of vitamin B12 per liter of said culture medium.

2. The process of claim 1, wherein culturing microalgae is conducted at a temperature of between 28 and 32°C.

3. The process of claims 1 or 2, wherein culturing microalgae is conducted at a temperature of 30°C.

4. The process of any one of claims 1 to 4, wherein it is added :

   ○ 0,1 mg to 200 mg of vitamin B1 per liter of said culture medium, and/or
   ○ 0,1 mg to 200 mg of vitamin B6 per liter of said culture medium.

5. The process of any one of claims 1 to 4, wherein microalgaes belonging to the Thraustochytriales family are selected from the group consisting of *Schizochytrium sp.* ATCC 20888, *Aurantiochytrium sp.* ATCC PRA 276 and *Schizochytrium sp.* deposited at the National Collection of Microorganism Cultures of Institut Pasteur under n°CNCM I-4469.

6. The process of any one of claims 1 to 5, wherein amounts of squalene produced are greater than or equal to 2g for 100g dry biomass, preferably between 2g and 12g for 100g dry biomass.

7. The process of any one of claims 1 to 6, wherein said culturing of microalgaes belonging to the Thraustochytriales sp. family is conducted by the following steps :

   - first, preculturing for 24 to 36 hours in Erlenmeyer flasks, at a temperature of 28°C from an isolated colony on an agar dish,
   - second, preculturing for 24 to 36 hours in Erlenmeyer flasks, at a temperature of 28°C with an inoculum of 1 % (v/v) resulting from the first preculturing,
   - culturing for 60 to 150 hours at 30°C, in a fermenter regulated to provide an oxygen transfer of at most 45 mmol/l/hour, with an inoculum of 0,5 to 2 % (v/v) resulting from the second preculturing.

8. The process of claim 7, wherein the addition of vitamins B12 is conducted in first and second preculturing steps, so that the total content of vitamins is between 1 and 10 $\mu$g/l of culture medium.

9. The process of claim 8, wherein vitamins B1 and B6 are also added to the first and second preculturing steps, so that the total content of vitamins B1 and B6 is between 100 and 200 $\mu$g/l of culture medium.

**10.** The process of claim 7, wherein the addition of vitamins B12 is conducted:

- in the first and second preculturing steps, so that the total content of vitamins is between 1 and 10 $\mu$g/l of culture medium,
- in the culturing step, so that the total content of vitamins is about 1000$\mu$g/l of culture medium.

**11.** The process of claim 10, wherein vitamins B1 and B6 are also added :

- in the first and second preculturing steps, so that the total content of vitamins is between 100 and 200 $\mu$g/l of culture medium,
- in the culturing step, so that the total content of vitamins is between 150 and 200 mg/l of culture medium.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2010023551 A

**Littérature non-brevet citée dans la description**

- **BHATTACHARJEE, P. et al.** *World J. Microb. Biotechnol.,* 2001, vol. 17, 811-816
- **LEWIS et al.** *Mar. Biotechnol.,* 2001, 439-447
- **JIANG et al.** *J. Agric. Food Chem.,* 2004, vol. 52, 1196-1200
- **QIAN LI et al.** *J. Agric. Food Chem.,* 2009, vol. 57, 4267-4272
- **LEWIS et al.** *Mar. Biotechnol.,* 2001, vol. 3, 439-447
- **G. CHEN et al.** *New Biotechnology,* 2010, vol. 27-4, 382-389
- **K. W. FAN et al.** *World J. Microbiol. Biotechnol.,* 2010, vol. 26-3, 1303-1309
- **C-J YUE ; Y. JIANG.** *Process Biochemisty,* 2009, vol. 44, 923-927